(19) Europäisches Patentamt
European Patent Office
Office européen des brevets

(11) Publication number: 0 175 544 A2

# (12) EUROPEAN PATENT APPLICATION

(21) Application number: 85306449.1

(22) Date of filing: 11.09.85

(51) Int. Cl.⁴: C 07 D 501/18

(30) Priority: 17.09.84 US 651012

(43) Date of publication of application: 26.03.86 Bulletin 86/13

(84) Designated Contracting States: AT BE CH DE FR GB IT LI LU NL SE

(71) Applicant: **ELI LILLY AND COMPANY, Lilly Corporate Center, Indianapolis Indiana 46285 (US)**

(72) Inventor: **Chou, Ta-Sen, 745 Canyon Road, Indianapolis Indiana 46217 (US)**
Inventor: **Hoyling, Richard Charles, 5514 Manker Street, Indianapolis Indiana 46227 (US)**
Inventor: **Lakin, Robert Eugene, 27 Fairwood Drive, Brownsburg Indiana 46112 (US)**
Inventor: **Yang, Kuo Shang, 340 Green Hill Court, Greenwood Indiana 46142 (US)**

(74) Representative: **Hudson, Christopher Mark et al, Erl Wood Manor, Windlesham Surrey GU20 6PH (GB)**

## (54) An improved process for cephalosporin intermediates.

(57) 7β-Amino-3-(1-pyridiniummethyl)-3-cephem-4-carboxylate dihydrochloride salt is obtained as the crystalline dihydrate directly from the N-deacylation reaction mixture by mixing said reaction mixture with acetonitrile, acetone or isopropyl alcohol, each containing 5% to 10% water.

EP 0 175 544 A2

ACTORUM AG

# AN IMPROVED PROCESS FOR CEPHALOSPORIN INTERMEDIATES

This invention relates to a process for the preparation of an intermediate for semi-synthetic cephalosporin antibiotics. In particular, it relates to an improved process for preparing 7-amino-3-(1-pyridiniummethyl)-3-cephem-4-carboxylate, hereinafter referred to as 3'-pyridinium-3-cephem nucleus, an intermediate for antibiotics, e.g. ceftazidime and cephaloridine.

Ceftazidime, (6R,7R)-7β-[(Z)-2-(2-amino-thiazol-4-yl)-2-(2-carboxyprop-2-yloxyimino)acetamido]-3-(1-pyridiniummethyl)-3-cephem-4-carboxylate, is described in U.S. Patent No. 4,258,041. One method for the preparation of ceftazidime comprises the N-acylation of the 3'-pyridinium-3-cephem nucleus represented by Formula (I):

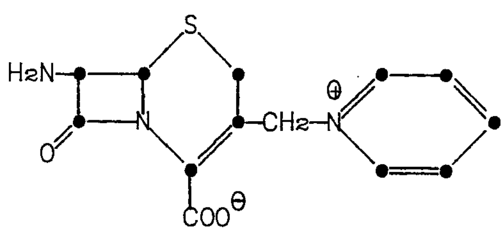

(I)

The 3'-pyridinium-3-cephem nucleus has been obtained as the crystalline dihydrochloride dihydrate by the method described in U.S. Patent No. 4,369,313.

Crystalline 7-amino-3-(1-pyridiniummethyl)-3-cephem-4-carboxylic acid dihydrochloride dihydrate may be obtained in high yields directly from the reaction mixture in which it is formed. The 3'-pyridinium-3-cephem nucleus $\cdot 2HCl \cdot 2H_2O$ is formed by the cleavage of a 7-substituted amino-3-(1-pyridiniummethyl)-3-cephem-4-carboxylate. For example, a 7-acylamino group is N-deacylated to provide the nucleus. The N-deacylation is accomplished by forming the imino chloride of the 7-position acylamino group and converting the imino chloride to the corresponding imino ether. The labile imino ether decomposes to the 7-amino nucleus compound. In a special case the N-formyl group of 7β-formamido-3-(1-pyridiniummethyl)-3-cephem-4-carboxylate is hydrolyzed in methanolic hydrochloric acid to provide the 3'-pyridinium-3-cephem nucleus. The 3'-pyridinium-3-cephem nucleus, formed in the cleavage reaction mixture, is obtained as the dihydrochloride dihydrate in crystalline form when the reaction mixture is diluted with acetonitrile containing about 5% to about 10% water or acetone containing about 5% to about 10% water, or isopropyl alcohol containing about 5% to about 10% water by volume. These ranges can be varied by 1 or 2% as indicated by the word "about".

According to the process of this invention, crystalline 7-amino-3-(1-pyridiniummethyl)-3-cephem-4-carboxylic acid dihydrochloride dihydrate is obtained directly from the crude reaction mixture in which it is formed by mixing the reaction mixture at a temperature of between about 0°C. and about 35°C. with a solvent system selected from acetonitrile, isopropyl alcohol or acetone, each containing between about 5% to 10% water by volume.

In particular, there is provided a process for preparing 7-amino-3-(1-pyridiniummethyl)-3-cephem-4-carboxylate dihydrochloride from the cleavage of a 7-substituted-amino-3-(1-pyridiniummethyl)-3-cephem-4-carboxylate characterized by mixing the cleavage reaction mixture containing the said 7-amino cleavage product with a solvent selected from acetonitrile, acetone or isopropyl alcohol, said solvent and/or reaction mixture in total containing from between about 5% and about 10% by volume of water relative to said solvent volume; and separating the 7-amino-3-(1-pyridiniummethyl)-3-cephem-4-carboxylate produced as the crystalline dihydrochloride dihydrate.

The 3'-pyridinium-3-cephem nucleus can be obtained by differing preparative routes. In general, a 7-substituted amino-3'-pyridinium-3-cephem-4-carboxylate is prepared by known methods such as nucleophilic displacement of a 7-substituted amino 3-acetoxymethyl- or 3-halomethyl-3-cephem-4-carboxylic acid or ester with pyridine. The 7-substituent group then is cleaved from the product to provide the 7-amino nucleus. The 7-substituted amino group may be a mono- or diacylated amino group, e.g. 2-thienylacetylamino, phenylacetylamino, phenoxyacetylamino, formylamino, phthalimido, succinimido, and like mono- and diacylated amino groups. The 7-substituted amino group also may be the amino group protected by a conventional amino-protecting group such as alkoxycarbonyl, cycloalkoxycarbonyl, bicyclicalkoxy carbonyl, or aralkoxycarbonyl group such as t-butyloxycarbonyl, ethoxycarbonyl, cyclopentyloxycarbonyl, adamantyloxycarbonyl, benzyloxycarbonyl,

p-nitrobenzyloxycarbonyl, and the like; a silylated amino group such as a trialkylsilyl group; or the trityl group. Following the formation of the 3'-pyridinium 7-substituted amino-3-cephem compound, the 7-substituent group is removed to provide the 3'-pyridinium-3-cephem nucleus. A 7-acylamino group of a 7-acylamino-3'-pyridinium compound is deacylated via the well known N-deacylation method comprising the formation of an imino chloride. In this method the acylamino portion of the 7-acylamino-3'-pyridinium compound is converted to the imino chloride with phosphorus pentachloride, and the latter is converted to the corresponding imino ether upon reaction with a lower alkanol, alkanediol, or benzyl alcohol. The imino ether is then decomposed to provide the 3'-pyridinium-3-cephem nucleus.

In an example of the overall process for preparing the 3'-pyridinium-3-cephem nucleus, 7-(2-thienylacetylamino)cephalosporanic acid is reacted with pyridine at about 60°C. in aqueous acetone to provide 7-(2-thienylacetylamino)-3-pyridiniummethyl-3-cephem-4-carboxylate, also known as cephaloridine. The 3'-pyridinium product is N-deacylated as follows. The compound is converted to the trimethylsilyl derivative in methylene chloride with trimethylchlorosilane in the presence of an acid-binding agent such as a dialkylaniline, and the derivative is reacted with phosphorus pentachloride at about -25°C. to -10°C. to form the imino chloride derivative. Next, the imino chloride is converted to the imino ether by addition of an alkanol or alkandiol, and the unstable imino ether decomposes to form the 3'-pyridinium-3-cephem nucleus.

In an alternative preparative method, 7-(2-thienyl-acetylamino)cephalosporanic acid is reacted with excess trimethylsilyl iodide to form the 3-iodomethyl derivative as the trimethylsilyl ester according to the method of Bonjouklian, U.S. Patent No. 4,266,049. The 3-iodomethyl derivative is reacted with pyridine to provide the pyridiniummethyl trimethylsilyl ester, and the latter is N-deacylated by the procedure described hereinabove to form the 3'-pyridinium-3-cephem nucleus compound.

The 3'-pyridinium-3-cephem nucleus can be prepared by the methods described above with 7-acyl-aminocephalosporanic acids or 3'-derivatives thereof other than the 2-thienylacetylaminocephalosporanic acid or derivatives thereof. For example, the 7-acyl group can be phenylacetyl, phenoxyacetyl or an alkanoyl group. One such alkanoyl group which can be used as a preferred starting material is 7-formamidocephalosporanic acid (N-formyl 7-ACA).

In practicing an embodiment of the process of this invention, N-formyl 7-ACA is reacted with excess trimethylsilyl iodide to form 7-formamido-3-iodomethyl-3-cephem-4-carboxylic acid trimethylsilyl ester. Without isolation, the 3-iodomethyl ester is reacted with pyridine to form the 7-formamido-3-pyridiniummethyl derivative. The latter then is hydrolyzed with a methanol solution of concentrated hydrochloric acid to provide in solution 7-amino-3-pyridiniummethyl-3-cephem-4-carboxylic acid dihydrochloride. The reaction mixture is then diluted with acetonitrile, acetone, or isopropyl alcohol in an amount corresponding to between about

one-third and about one-half of the volume of the reaction mixture. The water already present in the reaction mixture by virtue of the acid hydrolysis of the N-formyl group is sufficient to provide the amount of water to form the desired ratio of water to the diluting organic solvent of about 5% to about 10%. Accordingly, the organic solvent need not be diluted with water to the desired percentage in the instance where sufficient water is already present in the reaction mixture.

In the case in which the N-deacylation is carried out under substantially anhydrous conditions, e.g. N-deacylation via imino halide and imino ether formation, the reaction mixture is diluted with the aqueous organic solvent containing the desired ratio of water to solvent.

In an example of the process under the latter conditions, 7-(2-thienylacetylamino)-3-(1-pyridiniummethyl)-3-cephem-4-carboxylate is first silylated in methylene chloride with trimethylchlorosilane in the presence of a suitable base, e.g. a dialkylaniline. The silylated derivative is next reacted with phosphorus pentachloride at about -20°C. to about -25°C. to form the imino chloride and the latter is converted to the imino ether with methyl alcohol. After stirring the imino ether at room temperature to effect its decomposition to the pyridinium nucleus compound, the whole reaction mixture is poured into acetonitrile containing between 5% and 10% water. The pyridinium nucleus dihydrochloride dihydrate crystallizes from the diluted reaction mixture and is collected by filtration, decantation, centrifugation, or other suitable means.

In a further example of the process of this invention, 7-aminocephalosporanic acid is mixed in methylene chloride at a temperature between about 0°C. and about 5°C. with excess trimethylsilyl iodide in the presence of a dialkylaniline such as N,N-diethylaniline. The disilylated 3-iodomethyl derivative, 7-trimethylsilylamino-3-iodomethyl-3-cephem-4-carboxylic acid trimethylsilyl ester, formed in solution is treated with pyridine at a temperature about 15°C. to form the soluble disilylated 3'-pyridinium nucleus. The disilylated 3'-pyridinium nucleus is hydrolyzed by adding the reaction to a mixture of concentrated hydrochloric acid and methyl alcohol while maintaining the temperature of the hydrolysis mixture below about 25°C. After hydrolysis is complete, acetonitrile is then added with stirring to the hydrolysis mixture. The 7-amino-3-(1-pyridiniummethyl)-3-cephem-4-carboxylate dihydrochloride dihydrate crystallizes from the mixture and is collected by filtration or centrifugation. The crystalline product is dried at a temperature of about 40°C.

According to the invention, the 3'-pyridinium-3-cephem nucleus is isolated as the crystalline dihydrochloride dihydrate directly from the reaction mixture in which it is formed by the addition of a solvent system of the invention. The preferred solvent system is acetonitrile containing about 5% to about 10% water. The mode of addition is not critical; however, better crystallization often results when the 3'-pyridinium-3-cephem nucleus-containing reaction mixture is added to the aqueous solvent system. The reaction mixture is

diluted by using a volume of the aqueous solvent system corresponding to from about ¼ to about ½ of the volume of the reaction mixture.

The practice of this invention results in the direct recovery of crystalline product from the reaction mixture and, therefore, is especially economical for manufacturing purposes. More expensive recovery and crystallization procedures such as extraction, column chromatography, and recrystallization, therefore, can be avoided.

The aqueous solvent systems provided are also applicable to the purification of impure 3'-pyridinium-3-cephem nucleus ·2HCl and such use forms a further aspect of this invention. According to this aspect, non-crystalline 3'-pyridinium-3-cephem nucleus ·2HCl or impure nucleus $\cdot 2HCl \cdot 2H_2O$ is suspended in the organic solvent-water system of this invention and the suspension is treated with stirring with concentrated hydrochloric acid or hydrogen chloride. The amount of acid added is between about 0.05 mole and about 0.1 mole per mole of 3'-pyridinium-3-cephem nucleus dihydrochloride salt. The recrystallization is carried out at a temperature between about 5°C. and about 25°C. Preferably, the recrystallization is carried out in acetonitrile containing 5% to 10% water. The crystalline disalt dihydrate generally crystallizes a few minutes after acidification.

The following non-limiting Examples are provided to further illustrate the process of the invention.

## Example 1

To a 500 ml, three-necked flask were added 28.5 g of 7β-(2-thienylacetylamino)-3-(1-pyridiniummethyl)-3-cephem-4-carboxylate, 200 ml of methylene chloride, 35 ml of N,N-dimethylaniline, and 20 ml of trimethylchlorosilane. The mixture was stirred at room temperature for 30 minutes and the clear dark-amber solution was cooled to -25°C. To the cold solution were added with stirring 28.5 g of phosphorus pentachloride and the reaction mixture was stirred for one hour at a temperature between -20°C. to -25°C. Cold methyl alcohol (60 ml, -20°C.) was added in one portion to the reaction mixture. The temperature of the reaction mixture increased to 22°C. and was recooled to about 0°C. to -10°C. The mixture was stirred for two hours while the temperature increased to about 15 to 20°C. The reaction mixture was poured into 550 ml of acetonitrile containing 30 ml of water and 10 ml of methyl alcohol. The product formed as an oil on warming and started to crystallize. The mixture was cooled in an ice bath for 30 minutes and the crystalline 3'-pyridinium-3-cephem nucleus dihydrochloride dihydrate was filtered. The crystalline product was washed with 100 ml of acetonitrile-water, 4:1, v:v and dried overnight under vacuum. There were obtained 18.2 g of the nucleus (66.3% yield) as off-white crystals.

## Example 2

To a dry 100-gallon glass-lined still were added 165 l of methylene chloride and the solvent was

allowed to stand overnight under a slight nitrogen pressure. To the solvent were added 23.5 g of 7β-(2-thienylacetylamino)-3-(1-pyridiniummethyl)-3-cephem-4-carboxylate, 28.8 l of N,N-dimethylaniline (dried over sieves) and 16.5 l of trimethylchlorosilane. The still was then pressurized to 21 psi with nitrogen and cooled to -34°C. To the cold solution were added 23.5 kg of phosphorus pentachloride in three portions of 12 kg, 6 kg, and 5.5 kg with cooling after each addition to maintain the temperature about -20°C. to -25°C. The reaction mixture was stirred at -23°C. to -31°C. for one hour and a cold (ca. -5°C.) solution of 45.2 l of butanediol in 120 l of methylene chloride was added over three minutes. The temperature of the reaction mixture increased to 24°C. and stirring was continued for two hours at 16°C. to 20°C. The reaction mixture was added with stirring over 15 minutes to a second glass-lined still loaded with 415 l of acetonitrile containing 41.5 l of water. The still containing the reaction mixture was rinsed with 10 l of methylene chloride and the rinse added to the second still. The reaction mixture-acetonitrile mixture was evaporated under vacuum at a still temperature of 20°C. to 36°C. to reduce the volume by collecting 250 l of distillate. The still was then cooled with brine overnight and the crystalline 3'-pyridinium-3-cephem nucleus was collected by filtration. The nucleus was washed with 35 l of acetonitrile containing 1.75 l of water. The product was dried for eight days in an air oven at 35°C. There were obtained 15.06 kg (66.6% yield) of the crystalline 3'-pyridinium-3-cephem nucleus dihydrochloride dihydrate.

Example 3

To 120 l of methylene chloride contained in a nitrogen purged reactor were added 35 kg of 7β-formamido-3-acetoxymethyl-3-cephem-4-carboxylic acid and 11.76 kg of hexamethyldisilazane and the mixture heated to the reflux temperature for three hours. The solution (pH 7) obtained was cooled to 25°C. and added to a cold 5°C. solution of trimethylsilyl iodide in methylene chloride prepared in the following manner. To 60 l of methylene chloride were added 19.25 kg of iodine and 11.06 kg of hexamethyldisilazane and the mixture was heated at the reflux temperature for 30 minutes. The mixture was then cooled to 5°C. for use in the reaction. The reaction mixture was stirred for 70 minutes at -23°C. to form _in situ_ trimethylsilyl 7β-formamido-3-iodomethyl-3-cephem-4-carboxylate. To the reaction mixture were next added over two minutes 9.79 kg of pyridine and the mixture was stirred for about 196 minutes at 25°C. to form the 7β-formamido-3'-pyridinium compound. The reaction mixture was added to 65 l of methyl alcohol containing 35 l of concentrated hydrochloric acid and the mixture was stirred for two hours at 25°C. To the reaction mixture thus obtained and containing the 3'-pyridinium-3-cephem nucleus were added with stirring 85 l of acetonitrile and 1 g of crystalline 3'-pyridinium-3-cephem nucleus for seeding. An additional 85 l of acetonitrile were added over 75 minutes at 25°C. and the mixture was stirred overnight. The crystalline product was collected on a Buchner funnel and washed first with a solution of 15 l of methyl alcohol in 153 l of aceto-

nitrile and lastly with 70 l of acetonitrile. The crystalline 3'-pyridinium-3-cephem nucleus was dried for nine hours in a vacuum oven at 35°C. There were obtained 20.25 kg of the crystalline 3'-pyridinium-3-cephem nucleus (41.0% yield) as corrected by HPLC analysis.

## CLAIMS

1. A process for preparing 7-amino-3-(1-pyridiniummethyl)-3-cephem-4-carboxylate dihydrochloride from the cleavage of a 7-substituted-amino-3-(1-pyridiniummethyl)-3-cephem-4-carboxylate characterized by mixing the cleavage reaction mixture containing the said 7-amino cleavage product with a solvent selected from acetonitrile, acetone or isopropyl alcohol, said solvent and/or reaction mixture in total containing from between about 5% and about 10% by volume of water relative to said solvent volume; and separating the 7-amino-3-(1-pyridiniummethyl)-3-cephem-4-carboxylate produced as the crystalline dihydrochloride dihydrate.

2. A process as claimed in claim 1 in which the 7-substituted-amino cephem is a 7-acylamino cephem compound.

3. A process as claimed in claim 2 in which the 7-acylamino group is 2-thienylacetylamino, phenylacetylamino, phenoxyacetylamino, or formylamino.

4. A process as claimed in claim 3 in which 7-(2-thienylacetylamino)-3-(1-pyridiniummethyl)-3-cephem-4-carboxylate or 7-formylamino-3-(1-pyridiniummethyl)-3-cephem-4-carboxylate is N-deacylated.

5. A process as claimed in claim 1 in which the 7-substituted-amino cephem compound is a trialkylsilylamino cephem.

6. A process as claimed in claim 5 in which 7-trimethylsilylamino-3-(1-pyridiniummethyl)-3-cephem-4-carboxylic acid trimethylsilyl ester is cleaved by hydrolysis.

7. A process as claimed in claim 1 in which acetonitrile containing by volume between about 5% and about 10% of water is mixed with the cleavage reaction mixture.

8. A process as claimed in claim 1 in which the cleavage reaction mixture is mixed with a volume of solvent corresponding to between about ¼ and about ½ of the volume of said reaction mixture.

9. 7-amino-3-(1-pyridiniummethyl)-3-cephem-4-carboxylate dihydrochloride dihydrate whenever prepared according to a process as defined in any one of claims 1 to 8.

10. A process for preparing ceftazidime which comprises preparing 7-amino-3-(1-pyridiniummethyl)-3-cephem-4-carboxylate dihydrochloride dihydrate by a process as defined in any one of claims 1 to 8 and converting it to ceftazidime.